# EUROPÄISCHE PATENTSCHRIFT

(11) **EP 2 696 836 B1**
(45) Veröffentlichungstag und Bekanntmachung des Hinweises auf die Patenterteilung: **17.07.2019**
(21) Anmeldenummer: 12710661.5
(22) Anmeldetag: 16.03.2012
(51) Int. Cl.: A61J 1/20

(54) **VERWENDUNG EINER VORRICHTUNG UND EINES VERFAHRENS ZUR AUFBEREITUNG VON PHARMAZEUTISCHEN STOFFGEMISCHEN**
USE OF A DEVICE AND A METHOD FOR PREPARING PHARMACEUTICAL MATERIAL MIXTURES
UTILISATION D'UN DISPOSITIF ET D'UN PROCÉDÉ POUR PRÉPARER DES MÉLANGES DE SUBSTANCES PHARMACEUTIQUES

(30) Priorität: 12.04.2011 DE 102011016767
(43) Veröffentlichungstag der Anmeldung: 19.02.2014
(73) Patentinhaber: Sartorius Stedim Biotech GmbH, 37079 Göttingen (DE)
(72) Erfinder: JORNITZ, Maik, Manorville NY 11949 (US); SCHLACK, Stefan, 37083 Göttingen (DE)
(74) Vertreter: Stehl, Astrid
(86) Internationale Anmeldenummer: PCT/EP2012/001170
(87) Internationale Veröffentlichungsnummer: WO 2012/139694

(56) Entgegenhaltungen:
- WO-A1-2005/118031
- US-A- 4 219 038
- US-A- 5 592 940
- US-A- 5 609 572
- US-A- 5 941 867

## Beschreibung

### Gebiet der Erfindung

Die Erfindung betrifft die Verwendung einer Vorrichtung, bestehend aus einem in einer Aufnahme anordenbaren Aufbereitungsbehälter mit Ingredienzien, einem Vorratsbehälter mit einem Fluid, das über eine Zuleitung dem Aufbereitungsbehälter kontrolliert zuführbar ist, und einem Aufnahmebehälter, der über eine Ableitung mit dem Aufbereitungsbehälter korrespondiert.

Die Erfindung betrifft weiterhin die Verwendung eines Verfahrens mit folgenden Schritten:
a) Anordnen eines mit Ingredienzien vorkonfektionierten Aufbereitungsbehälters in einer Aufnahme einer Vorrichtung,
b) Zuführung eines Fluides aus einem Vorratsbehälter über eine Zuleitung in den Aufbereitungsbehälter,
c) Aufbereitung der Ingredienzien mit dem Fluid zu einem Stoffgemisch, und
d) Abführen des Stoffgemisches aus dem Aufbereitungsbehälter über eine Ableitung in einen Aufnahmebehälter.

Die Erfindung betrifft weiterhin eine Vorrichtung, bestehend aus einem in einer Aufnahme anordenbaren Aufbereitungsbehälter mit Ingredienzien, einem Vorratsbehälter mit einem Fluid, das über eine Zuleitung dem Aufbereitungsbehälter kontrolliert zuführbar ist, und einem Aufnahmebehälter, der über eine Ableitung mit dem Aufbereitungsbehälter korrespondiert,

### Stand der Technik

Im pharmazeutischen und biopharmazeutischen Bereich, insbesondere im vorklinischen oder klinischen Bereich, sowie in Forschung und Entwicklung ist es häufig notwendig, kleine Volumen von Puffern und Medien aufzubereiten. Die Puffer und Medien werden oft vorkonfektioniert nach vorbestimmten Rezepturen aufbereitet, die einer sorgfältigen und relativ zeitaufwendigen Behandlung durch das Fachpersonal bedürfen. Von besonderer Bedeutung ist es dabei, Stoffgemische aufzubereiten, die auf die vom Endverbraucher benötigten Volumina, Konzentrationen und Komponenten bzw. Medien abgestimmt sind.

Beispielsweise ist aus der WO 2010/125329 A1 eine Vorrichtung und ein Verfahren zur portionsweisen Aufbereitung von Getränken, insbesondere zur Aufbereitung bzw. Aufbrühen von Kaffee bekannt. Diese Vorrichtung besteht aus einem in einer Aufnahme der bekannten Vorrichtung anordenbaren Aufbereitungsbehälter mit Ingredienzien, nämlich Kaffeepulver, einem Vorratsbehälter mit einem Fluid, nämlich Wasser, das über eine Zuleitung dem Aufbereitungsbehälter kontrolliert zugeführt wird. Das Wasser, das dem Aufbereitungsbehälter in erhitzter Form zugeführt wird, brüht die Kaffeepulvermischung auf, die als aufbereitetes Stoffgemisch, nämlich als Kaffee, über eine Ableitung einem Aufnahmebehälter, nämlich einer Kaffeetasse, zugeführt wird.

Der Aufbereitungsbehälter ist dabei als eine verschlossene Kapsel bzw. Kartusche ausgebildet, die das aufzubereitende Stoffgemisch beinhaltet.

Es sind aber auch Vorrichtungen bekannt, die anstelle einer Kapsel oder Kartusche ein sogenanntes Pad verwenden.

Ein Beispiel zur Aufbereitung von pharmazeutischen Stoffgemischen ist bekannt durch US5941867.

### Aufgabenstellung

Aufgabe der vorliegenden Erfindung ist es, eine Vorrichtung und ein Verfahren zur Verfügung zu stellen, mit dem die Aufbereitung von pharmazeutischen oder biopharmazeutischen Stoffgemischen auch mit vorgegebenen kleinen bzw. mittleren Volumina, Konzentrationen und Medien bzw. Komponenten einfach und sicher auf den Endverbraucher abgestimmt, ermöglicht werden kann.

### Darstellung der Erfindung

Diese Aufgabe wird gelöst durch die Verwendung einer Vorrichtung, bestehend aus einem in einer Aufnahme anordenbaren Aufbereitungsbehälter mit Ingredienzien, einem Vorratsbehälter mit einem Fluid, das über eine Zuleitung dem Aufbereitungsbehälter kontrolliert zuführbar ist, und einem Aufnahmebehälter, der über eine Ableitung mit dem Aufbereitungsbehälter korrespondiert, zur Aufbereitung von pharmazeutischen oder biopharmazeutischen Stoffgemischen.

Unter Vorratsbehälter soll auch ein Fluidaufbereitungssystem und, soweit es sich bei dem Fluid um Wasser handelt, soll auch ein Wasseraufbereitungssystem für analysenreines Wasser, wie es beispielsweise von der Sartorius AG in Göttingen unter der Bezeichnung Arium® vertrieben wird, verstanden werden.

Dem Fachmann hat es bisher ferngelegen, Vorrichtungen und Verfahren, wie sie zur Aufbereitung von Getränken, wie z.B. Kaffee, bekannt sind, für die Aufbereitung von pharmazeutischen oder biopharmazeutischen Stoffgemischen, die unter hochreinen und sterilen Bedingungen erfolgen muss, in Betracht zu ziehen.

Durch die Adaption der an sich bekannten Vorrichtungen für die Verwendung bzw. zur Aufbereitung von pharmazeutischen oder biopharmazeutischen Stoffgemischen wird es ermöglicht, auch unter hochreinen und sterilen Bedingungen pharmazeutische oder biopharmazeutische Stoffgemische relativ schnell, sicher und einfach aus kleinen bis mittleren Mengen bzw. Volumina, in vorgebbaren Konzentrationen und Medien bzw. Komponenten, die auf den Endverbraucher abgestimmt sind, aufzubereiten. Grundsätzlich ist diese Vorrichtung auch für größere Mengen bzw. Volumina geeignet.

Dabei erfolgt gemäß einer bevorzugten Ausführungsform der Erfindung die Verwendung der Vorrichtung zur Aufbereitung von patientenindividuellen Medikamenten.

Nach einer weiteren bevorzugten Ausführungsform der Verwendung weist die Vorrichtung in der Zuleitung zum Aufbereitungsbehälter und/oder in der Ableitung einen Sterilfilter oder einen Virusfilter oder eine Kombination von Sterilfilter und Virusfilter auf. Dadurch wird sichergestellt, dass dem Aufbereitungsbehälter nur steriles und/oder virusfreies Fluid bzw. dem Aufnahmebehälter nur steriles und/oder virusfreies aufbereitetes Stoffgemisch zugeführt wird.

Nach einer weiteren bevorzugten Ausführungsform der Erfindung ist der Aufbereitungsbehälter als eine Kartusche bzw. eine Kapsel ausgebildet. Eine Kartusche bzw. Kapsel kann dabei sowohl die Zuleitung als auch die Ableitung auf einer Seite aufweisen. Es ist aber auch möglich, als Aufbereitungsbehälter ein Pad bzw. Membranpad zu verwenden. Bei der Verwendung eines Membranpads sind Zuleitung und Ableitung aufeinander gegenüberliegenden Seiten des Pads angeordnet.

Auch ist es möglich, dem Aufbereitungsbehälter in der Vorrichtung aus einer Mehrzahl von Vorratsbehältern individuell Ingredienzien zuzuführen. Grundsätzlich ist es auch möglich, zwei oder mehr Aufbereitungsbehälter in einer Parallelschaltung nebeneinander oder in einer Reihenschaltung hintereinander anzuordnen. Über Schlauchverbindungen, die beispielsweise steril verschweißt werden, können die Aufbereitungsbehälter miteinander verbunden werden

Gemäß einer weiteren bevorzugten Ausführungsform der Erfindung weist der Aufbereitungsbehälter eine Mischanordnung auf, die den Aufbereitungsvorgang erleichtert. Die Mischanordnung kann dabei auch durch Mischdüsen gebildet werden. Grundsätzlich kann der Aufbereitungsbehälter oder auch der Aufnahmebehälter auch in einer Rüttelvorrichtung zur Erzeugung eines Mischvorganges gerüttelt werden.

Die Aufgabe bezüglich der Vorrichtung wird weiterhin in Verbindung mit dem Oberbegriff des Anspruches 11 dadurch gelöst, dass zur Aufbereitung von pharmazeutischen oder biopharmazeutischen Stoffgemischen dem Aufbereitungsbehälter ein Steril- bzw. Virusfilter oder eine Kombination daraus vorgelagert oder nachgelagert ist.

Diese Vorrichtung weist die oben beschriebenen Vorteile auf.

Gemäß einer bevorzugten Ausführungsform der Erfindung weist der Aufbereitungsbehälter mindestens einen Steril- bzw. Virusfilter oder eine Kombination daraus auf. Diese(r) ist bzw. sind an Ein- und/oder Austrittsöffnung(en) angeordnet, innerhalb und/oder außerhalb des Aufbereitungsbehälters.

Dabei kann der Aufbereitungsbehälter auch als ein Pad ausgebildet sein, an oder in dem ein Sterilfilter angeordnet ist.

Nach einer weiteren bevorzugten Ausführungsform der Erfindung sind der Aufbereitungsbehälter und der Aufnahmebehälter über eine Schlauchverbindung miteinander verbunden bzw. miteinander, beispielsweise durch eine sterile Verschweißung, verbindbar. So könnte der Aufbereitungsbehälter ausgangsseitig und der Aufnahmebehälter (beispielsweise ein Beutel) eingangsseitig jeweils einen verschweißten Schlauch aufweisen, deren freie Enden miteinander in einer Schweißeinheit steril verschweißt werden.

Gemäß einer weiteren bevorzugten Ausführungsform der Erfindung weist der Aufbereitungsbehälter und/oder der Aufnahmebehälter mindestens einen Sensor zur Bestimmung eines Parameters des aufbereiteten Stoffgemisches auf. Dabei können der pH-Wert, die Temperatur, die Leitfähigkeit oder andere Parameter gemessen und gegebenenfalls über die Steuer- und Kontrolleinheit geregelt werden. Auch ist es möglich, einen Biochip, beispielsweise zur Sterilitätsbestimmung, im Aufbereitungsbehälter und/oder Aufnahmebehälter anzuordnen.

Die Aufgabe bezüglich des Verfahrens wird gelöst durch die Verwendung eines Verfahrens mit folgenden Schritten:
a) Anordnen eines mit Ingredienzien vorkonfektionierten Aufbereitungsbehälters in einer Aufnahme einer Vorrichtung
b) Zuführung eines Fluids aus einem Vorratsbehälter über eine Zuleitung in den Aufbereitungsbehälter,
c) Aufbereitung der Ingredienzien mit dem Fluid zu einem Stoffgemisch, und
d) Abführen des Stoffgemisches aus dem Aufbereitungsbehälter über eine Ableitung in einen Aufnahmebehälter, zur Aufbereitung von pharmazeutischen oder biopharmazeutischen Stoffgemischen.

Durch die Adaption des an sich bekannten Verfahrens für die Verwendung bzw. zur Aufbereitung von pharmazeutischen oder biopharmazeutischen Stoffgemischen wird es ermöglicht, auch unter hochreinen und sterilen Bedingungen pharmazeutische oder biopharmazeutische Stoffgemische relativ schnell, sicher und einfach in kleinen bzw. mittleren Mengen bzw. Volumina, in vorgebbaren Konzentrationen und Medien bzw. Komponenten, die auf den Endverbraucher abgestimmt sind, aufzubereiten.

Die Zuleitung kann weiterhin einen Filter, beispielsweise einen Sterilfilter beinhalten. Ebenso kann das Abführen des Stoffgemisches über einen derartigen Filter geschehen.

Der vorkonfektionierte Aufbereitungsbehälter ist gegenüber seiner Umwelt abgeschlossen und wird erst in der Aufnahme der Vorrichtung, beispielsweise durch Aufstechen mit einen Einstechdorn, mit der Zuleitung und der Ableitung verbunden.

Durch die Verwendung des vorkonfektionierten Aufbereitungsbehälters wird die richtige Dosierung der Ingredienzien sichergestellt.

Die Aufgabe bezüglich des Verfahrens wird weiterhin gelöst durch die Verwendung eines Verfahrens mit folgenden Schritten:
a) Anordnen eines Aufbereitungsbehälters in einer Aufnahme einer Vorrichtung,
b) Zuführung von Ingredienzien aus einer Mehrzahl von Vorratsbehältern in das Aufbereitungsbehältnis,
c) Zuführung eines Fluides aus einem Vorratsbehälter über eine Zuleitung in den Aufbereitungsbehälter,
d) Aufbereitung der Ingredienzien mit dem Fluid zu einem Stoffgemisch, und
e) Abführen des Stoffgemisches aus dem Aufbereitungsbehälter über eine Ableitung in einen Aufnahmebehälter zur Aufbereitung von pharmazeutischen oder biopharmazeutischen Stoffgemischen.

Durch die Zuführung von Ingredienzien aus einer Mehrzahl von Vorratsbehältern in das Aufbereitungsbehältnis ist eine individuelle Aufbereitung mit unterschiedlichen Komponenten bzw. eine variierbare Zusammensetzung des Stoffgemisches bei Verwendung des gleichen Aufbereitungsbehälters möglich.

Gemäß einer bevorzugten Ausführungsform der Erfindung werden die Verfahren zur Aufbereitung von patientenindividuellen Medikamenten, wie beispielsweise Infusionen oder Zytostatika, oder Parenteralia verwendet. Ebenso ist eine Aufbereitung von Kunstblut, synthetischem Blut oder Blutersatz wie beispielsweise hämoglobinbasierten Blutersatzmitteln oder Perfluorcarbonen möglich.

Nach einer weiteren bevorzugten Ausführungsform der Erfindung wird nach Einsetzen des Aufbereitungsbehälters von einer Leseeinheit eine Codierung des Aufbereitungsbehälters ausgelesen und aus einem Speicher die zur Durchführung des Verfahrens notwendigen Parameter aufgerufen und einer Kontroll- und Steuereinheit zur Verfügung gestellt. Damit erfolgt eine automatische Aufbereitung des Stoffgemisches, die einfach und sicher ist und zugleich eine individuelle Zusammensetzung ermöglicht. Ebenso ist eine Identifizierung mit Hilfe elektromagnetischer Wellen (RFID) oder anderen Identifikationsmechanismen ausführbar.

Weitere Einzelheiten der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibung der Zeichnungen

In den Zeichnungen zeigen:
- Figur 1:: eine schematische Darstellung einer Vorrichtung zur Aufbereitung von pharmazeutischen oder biopharmazeutischen Stoffgemischen,

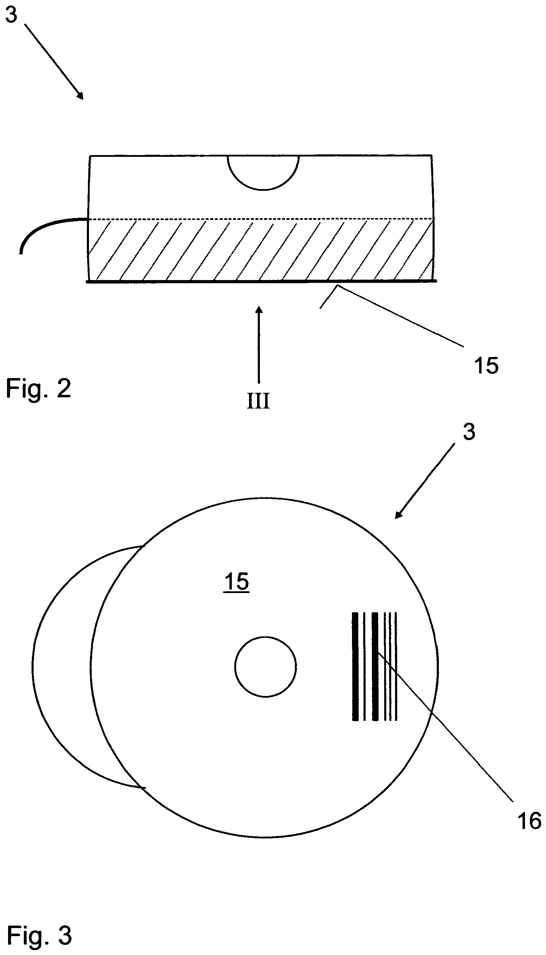

- Figur 2:: eine Seitenansicht eines Aufbereitungsbehälters,
- Figur 3:: eine Untersicht unter den Behälter von Figur 2 aus Richtung III,
- Figur 4:: eine schematische Darstellung einer weiteren Vorrichtung zur Aufbereitung von pharmazeutischen oder biopharmazeutischen Stoffgemischen mit einem Sterilfilter in der Ableitung des Aufbereitungsbehälters, und
- Figur 5:: eine weitere schematische Darstellung einer Vorrichtung zur Aufbereitung von pharmazeutischen oder biopharmazeutischen Stoffgemischen mit einer Mehrzahl von Vorratsbehältern.

### Beschreibung der Ausführungsbeispiele

Eine Vorrichtung 1 besteht im Wesentlichen aus einer Aufnahme 2 für einen Aufbereitungsbehälter 3, einem Vorratsbehälter 4 und einer Steuer- und Kontrolleinheit 5.

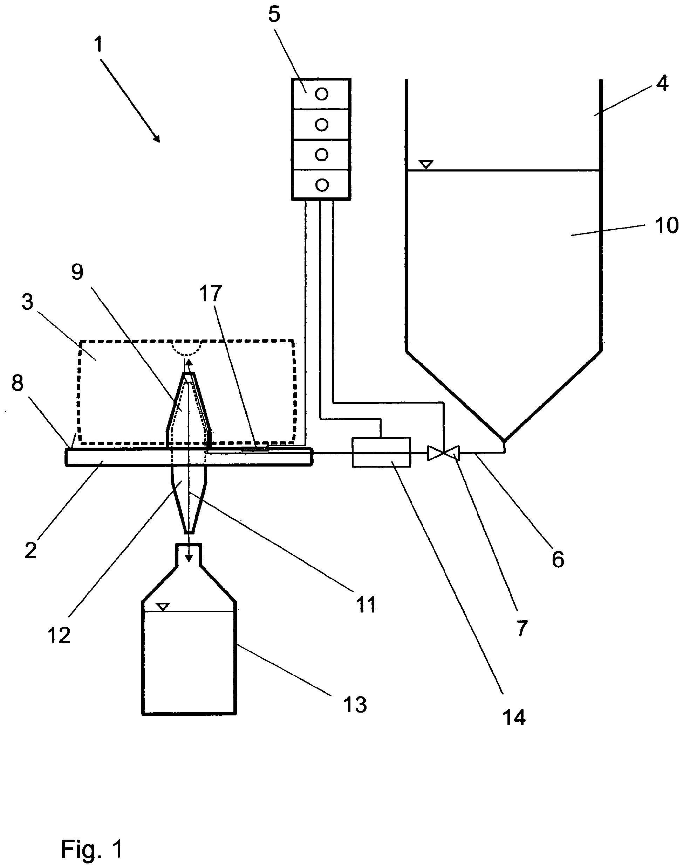

Entsprechend dem Ausführungsbeispiel der Figur 1 ist die Aufnahme 2 über eine Zuleitung 6 mit dem Vorratsbehälter 4 verbunden. In der Zuleitung 6 ist ein von der Steuer- und Kontrolleinheit 5 kontrolliertes Regelventil 7 angeordnet. Die Aufnahme 2 weist auf ihrer dem Aufbereitungsbehälter 3 zugewandten Oberseite 8 einen Einstechdorn 9 auf. Nach dem Aufsetzen des Aufbereitungsbehälters 3 auf die Aufnahme 2 ist über den Einstechdorn 9 zum einen ein in dem Vorratsbehälter 4 befindliches Fluid 10 dem Aufbereitungsbehälter 3 zuführbar und zum anderen als aufbereitetes Stoffgemisch 11 über eine mit dem Einstechdorn 9 verbundene Ableitung in einen Aufnahmebehälter 13 ableitbar. Das dem Aufbereitungsbehälter 3 zuzuführende Fluid 10 kann in einer Temperiereinrichtung 14 von der Steuer- und Kontrolleinheit 5 kontrolliert und temperiert werden. Der Aufbereitungsbehälter 3 weist auf seiner der Oberseite 8 der Aufnahme 2 zugewandten Unterseite 15 eine Codierung 16 auf, die beispielsweise als ein Barcode ausgebildet ist, der von einer Leseeinheit 17 ausgelesen und der Steuer- und Kontrolleinheit 5 zugeführt wird. Die Codierung 16 wird von der Steuer- und Kontrolleinheit genutzt, um aus einem nicht weiter dargestellten Speicher, die zur Durchführung des Verfahrens notwendigen Parameter aufzurufen und diese zur Durchführung des Aufbereitungsverfahrens zu nutzen.

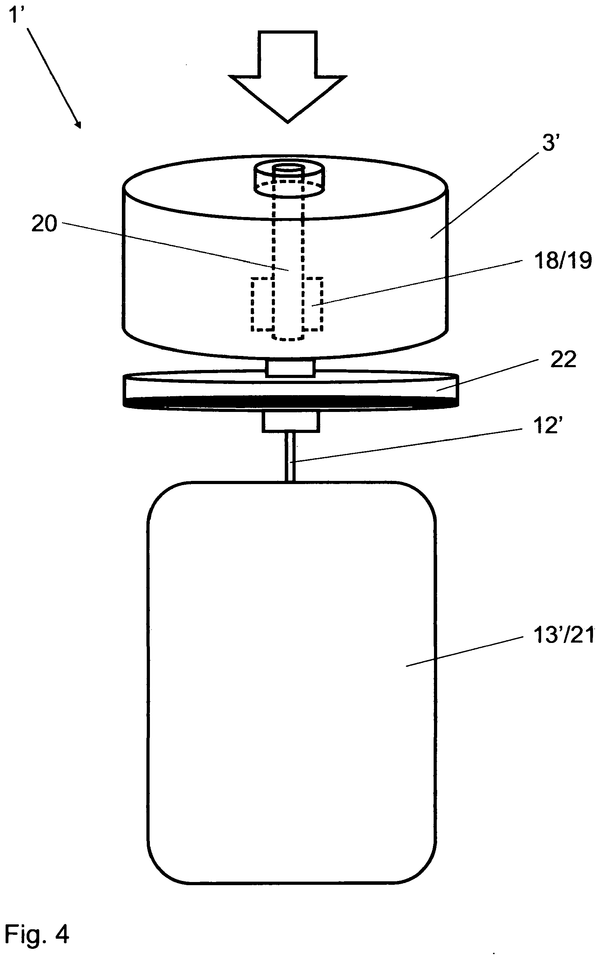

Entsprechend dem Ausführungsbeispiel der Figur 4, die eine Vorrichtung 1' zeigt, deren Aufbereitungsbehälter 3' in seinem Inneren eine Mischanordnung 18 aufweist, die als ein Rührer 19 mit einem Rührschaft 20 ausgebildet ist, über den Fluid oder Ingredienzien zuführbar sind. In der Ableitung 12' ist dem Aufnahmebehälter 13', der als ein Einwegbeutel 21 ausgebildet ist, ein Sterilfilter 22 vorgelagert.

Damit kann ein Verfahren beispielsweise mit folgenden Schritten durchgeführt werden:
a) Anordnen des Aufbereitungsbehälters 3' in der Aufnahme der Vorrichtung 1',
b) kontrollierte automatische Zuführung von Ingredienzien sowie eines Fluides (bspw. Wasser) 10 aus entsprechenden Vorratsbehältern in den Aufbereitungsbehälter 3'),
c) Aufbereitung der Ingredienzien mit dem Fluid zu einem Stoffgemisch, wobei durch die Mischanordnung 18 bzw. deren Rührer 19 das Stoffgemisch durchmischt wird,
d) Messen von vorgegebenen Parametern (beispielsweise Leitfähigkeit, pH-Wert und Temperatur) und Freigabe
e) Abführen des Stoffgemisches aus dem Aufbereitungsbehälter 3' über den Sterilfilter 22 (beispielsweise durch Durchbrechen einer Barriere) und über die Ableitung 12' in den Aufnahmebehälter13',
f) Ausdrucken eines Etikettes und aufkleben auf den Aufbereitungsbehälter, und
g) Verschließen des Aufbereitungsbehälters 3' bzw. des Einwegbeutels 21 (zum Beispiel durch verschweißen und abtrennen des Ableitungsschlauches 12').

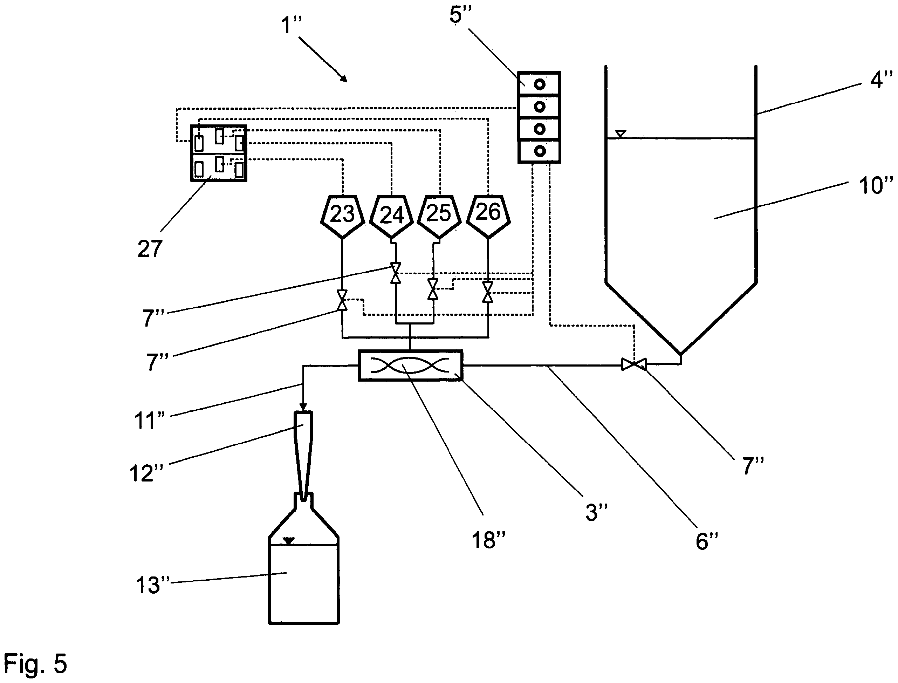

Entsprechend dem Ausführungsbeispiel der Figur 5 weist die Vorrichtung 1'' einen Aufbereitungsbehälter 3" mit einer Mischanordnung 18" auf. Dem Aufbereitungsbehälter 3" sind eine Mehrzahl von Vorratsbehältern 23, 24, 25, 26, im Ausführungsbeispiel 4, vorgelagert, über die unterschiedliche Medien bzw. Ingredienzien von der Steuer- und Kontrolleinheit 5" und zugeordneten Regelventilen 7" zugeführt werden können. Das Fluid 10" aus dem Vorratsbehälter 4" wird aus dem Aufbereitungsbehälter 3" ebenfalls über ein Regelventil 7" zugeführt. Entsprechend wird das aufbereitete Stoffgemisch 1" über eine Ableitung 12" einem Aufnahmebehälter 13" zugeführt. Die Vorratsbehälter 23, 24, 25, 26 können sowohl über die Steuer- und Kontrolleinheit 5" als auch über einen Touchscreen 27 manuell ausgewählt werden.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum an Variationsmöglichkeiten an die Hand gegeben.

### Bezugszeichenliste

- 1, 1', 1": Vorrichtung
- 2: Aufnahme
- 3, 3', 3": Aufbereitungsbehälter
- 4, 4": Vorratsbehälter
- 5, 5": Steuer- und Kontrolleinheit
- 6: Zuleitung
- 7, 7": Regelventil
- 8: Oberseite von 2
- 9: Einsteckdorn
- 10, 10": Fluid
- 11, 11": Stoffgemisch
- 12, 12', 12": Ableitung
- 13, 13', 13": Aufnahmebehälter
- 14: Temperiereinrichtung
- 15: Unterseite von 3
- 16: Codierung von 3
- 17: Leseeinheit
- 18, 18": Mischanordnung von 3'
- 19: Rührer von 18
- 20: Hohlschaft von 19
- 21: Einwegbeutel von 13'
- 22: Sterilfilter
- 23: Vorratsbehälter
- 24: Vorratsbehälter
- 25: Vorratsbehälter
- 26: Vorratsbehälter
- 27: Touchscreen

## Patentansprüche

1. Verwendung einer Vorrichtung (1, 1', 1"), bestehend aus einem in einer Aufnahme (2) anordenbaren Aufbereitungsbehälter (3, 3', 3") mit Ingredienzien, einem Vorratsbehälter (4,4") mit einem Fluid (10, 10"), das über eine Zuleitung (6) dem Aufbereitungsbehälter (3, 3', 3") kontrolliert zuführbar ist, und einem Aufnahmebehälter, der über eine Ableitung (12, 12', 12") mit dem Aufbereitungsbehälter (3, 3', 3") korrespondiert, zur Aufbereitung von pharmazeutischen oder biopharmazeutischen Stoffgemischen (11, 11").

2. Verwendung nach Anspruch 1, zur Aufbereitung von patientenindividuellen Medikamenten.

3. Verwendung nach Anspruch 1 oder 2, wobei die Vorrichtung in der Zuleitung (6) zum Aufbereitungsbehälter (3') und/oder in der Ableitung (12') einen Sterilfilter (22), oder einen Virusfilter oder eine Kombination von Sterilfilter und Virusfilter aufweist.

4. Verwendung nach einem der Ansprüche 1 bis 3, wobei der Aufbereitungsbehälter (3, 3', 3") als eine Kartusche oder ein Pad ausgebildet ist.

5. Verwendung nach einem der Ansprüche 1 bis 4, wobei dem Aufbereitungsbehälter (3") in der Vorrichtung (1") aus einer Mehrzahl von Vorratsbehältern (23, 24, 25, 26) Ingredienzien zuführbar sind.

6. Verwendung nach einem der Ansprüche 1 bis 5, wobei der Aufbereitungsbehälter (3', 3") eine Mischanordnung (18, 18") aufweist.

7. Verwendung eines Verfahrens mit folgenden Schritten.
a) Anordnen eines mit Ingredienzien vorkonfektionierten Aufbereitungsbehälters (3, 3', 3") in einer Aufnahme (2) einer Vorrichtung (1, 1', 1"),
b) Zuführen eines Fluids (10) aus einem Vorratsbehälter (4, 4") über eine Zuleitung (6) in den Aufbereitungsbehälter (3, 3"),
c) Aufbereitung der Ingredienzien mit dem Fluid (10, 10") zu einem Stoffgemisch (11, 11"), und
d) Abführen des Stoffgemisches (11, 11") aus dem Aufbereitungsbehälter (3, 3', 3") über eine Ableitung (12, 12', 12") in einen Aufnahmebehälter (13, 13', 13"), zur Aufbereitung von pharmazeutischen oder biopharmazeutischen Stoffgemischen (11, 11").

8. Verwendung nach Anspruch 7 zur Aufbereitung von patientenindividuellen Medikamenten.

9. Verwendung nach einem der Ansprüche 7 bis 8, wobei nach Einsetzen des Aufbereitungsbehälters (3) von einer Leseeinheit (17) eine Codierung (16) des Aufbereitungsbehälters (3) ausgelesen und aus einem Speicher die zur Durchführung des Verfahrens notwendigen Parameter aufgerufen und einer Kontroll- und Steuereinheit (5) zur Verfügung gestellt werden.

10. Vorrichtung (1, 1', 1"), bestehend aus einem in einer Aufnahme (2) anordenbaren Aufbereitungsbehälter (3, 3', 3") mit Ingredienzien , einem Vorratsbehälter (4, 4") mit einem Fluid (10, 10"), das über eine Zuleitung (6) dem Aufbereitungsbehälter (3, 3', 3") kontrolliert zuführbar ist, und einem Aufnahmebehälter, der über eine Ableitung (12, 12', 12") mit dem Aufbereitungsbehälter (3, 3', 3") korrespondiert, **dadurch gekennzeichnet, dass** zur Aufbereitung von pharmazeutischen oder biopharmazeutischen Stoffgemischen (11, 11") dem Aufbereitungsbehälter (3, 3', oder 3") ein Sterilfilter (22), oder ein Virusfilter oder eine Kombination von Sterilfilter und Virusfilter, vorgelagert oder nachgelagert ist.

11. Vorrichtung nach Anspruch 11, **dadurch gekennzeichnet, dass** der Aufbereitungsbehälter (3, 3', 3") mindestens einen Sterilfilter (22) oder einen Virusfilter oder eine Kombination von Sterilfilter und Virusfilter aufweist.

12. Vorrichtung nach Anspruch 11 oder 12, **dadurch gekennzeichnet, dass** der Aufbereitungsbehälter (3, 3', 3") als ein Pad ausgebildet ist an oder in dem ein Sterilfilter oder Virusfilter oder eine Kombination von Sterilfilter und Virusfilter angeordnet ist.

13. Vorrichtung nach einem der Ansprüche 11 bis 13, **dadurch gekennzeichnet, dass** der Aufbereitungsbehälter (3, 3', 3") und der Aufnahmebehälter (13, 13', 13") über eine Schlauchverbindung miteinander verschweissbar sind.

14. Vorrichtung nach einem der Ansprüche 11 bis 14, **dadurch gekennzeichnet, dass** der Aufbereitungsbehälter (3, 3', 3") und/oder der Aufnahmebehälter (13, 13', 13") mindestens einen Sensor zur Bestimmung eines Parameters des aufbereiteten Stoffgemisches aufweist.

## Claims

1. Use of a device (1, 1', 1") consisting of a preparation container (3, 3', 3") which can be arranged in a receiver (2), with ingredients, a reservoir (4, 4") with a fluid (10, 10"), which can be fed in controlled manner via a feed line (6) to the preparation container (3, 3', 3"), and a receiving container, which co-operates with the preparation container (3, 3', 3") by way of a drain line (12, 12', 12"), for preparation of pharmaceutical or biopharmaceutical substance mixtures (11, 11").

2. Use according to claim1, for preparation of patient-specific medicaments.

3. Use according to claim 1 or 2, wherein the device comprises a sterile filter (22) or a virus filter or a combination of sterile filter and virus filter in the feed line (6) to the preparation container (3') and/or in the drain line (12').

4. Use according to any one of claims 1 to 3, wherein the preparation container (3, 3', 3") is constructed as a cartridge or a pad.

5. Use according to any one of claims 1 to 4, wherein ingredients can be fed to the preparation container (3") in the device (1) from a plurality of reservoirs (23, 24, 25, 26).

6. Use according to any one of claims 1 to 5, wherein the preparation container (3', 3") comprises a mixing arrangement (18, 18").

7. Use of a method with the following steps:
a) arranging a preparation container (3, 3', 3"), which is pre-furnished with ingredients, in a receiver (2) of a device (1, 1', 1"),
b) feeding a fluid (10) from a reservoir (4, 4") into the preparation container (3, 3") via a feed line (6),
c) preparing the ingredients with the fluid (10, 10") to form a substance mixture (11, 11") and
d) discharging the substance mixture (11, 11") from the preparation container (3, 3', 3") via the drain line (12, 12', 12") into a receiving container (13, 13', 13"), for preparation of pharmaceutical or biopharmaceutical substance mixtures (11, 11").

8. Use according to claim 7 for preparing patient-specific medicaments.

9. Use according to one of claims 7 and 8, wherein after insertion of the preparation container (3) a code (16) of the preparation container (3) is read out by a reader (17), the parameters necessary for performance of the method are called up from a memory, and a checking and controlling unit (5) is provided.

10. Device (1, 1', 1") consisting of a preparation container (3, 3', 3"), which can be arranged in a receiver (2), with ingredients, a reservoir (4, 4") with a fluid (10, 10"), which can be fed in controlled manner by way of a feed line (6) to the preparation container (3, 3', 3"), and a receiving container, which co-operates with the preparation container (3, 3', 3") by way of a drain line (12, 12', 12"), **characterised in that** for preparation of pharmaceutical or biopharmaceutical substance mixtures (11, 11") a sterile filter (22) or a virus filter or a combination of sterile filter and virus filter is arranged upstream or downstream of the preparation container (3, 3' or 3").

11. Device according to claim 11, **characterised in that** the preparation container (3, 3', 3") comprises at least one sterile filter (22) or a virus filter or combination or sterile filter and virus filter.

12. Device according to claim 11 or 12, **characterised in that** the preparation container (3, 3', 3") is constructed as a pad at or in which a sterile filter or virus filter or a combination of sterile filer and virus filter is arranged.

13. Device according to one of claims 11 and 12, characterised I that the preparation container (3, 3', 3") and the receiving container (13, 13', 13") can be bonded together by way of a hose connection.

14. Device according to any one of claims 11 to 13, **characterised in that** preparation container (3, 3', 3") and/or the receiving container (13, 13', 13") comprises or comprise at least one sensor for determining a parameter of the substance mixture to be prepared.

## Revendications

1. Utilisation d'un dispositif (1, 1', 1"), constitué d'un contenant de préparation (3, 3', 3") pouvant être agencé dans un logement (2) et renfermant des ingrédients, d'un contenant de stockage (4, 4") renfermant un fluide (10, 10"), qui peut être amené de façon régulée au contenant de préparation (3, 3', 3") par l'intermédiaire d'une conduite d'amenée, et d'un contenant de réception, qui communique avec le contenant de préparation (3, 3', 3") par l'intermédiaire d'une conduite d'évacuation (12, 12', 12"), pour la préparation de mélanges de substances pharmaceutiques ou biopharmaceutiques (11, 11").

2. Utilisation selon la revendication 1, pour la préparation de médicaments adaptés individuellement à un patient.

3. Utilisation selon la revendication 1 ou 2, dans laquelle le dispositif présente dans la conduite d'amenée (6) menant au contenant de préparation (3') et/ou dans la conduite d'évacuation (12') un filtre stérile (22), ou un filtre antiviral ou une combinaison d'un filtre stérile et d'un filtre antiviral.

4. Utilisation selon l'une quelconque des revendications 1 à 3, dans laquelle le contenant de préparation (3, 3', 3") est réalisé sous la forme d'une cartouche ou d'un tampon.

5. Utilisation selon l'une quelconque des revendications 1 à 4, dans laquelle des ingrédients peuvent être amenés d'une pluralité de contenants de stockage (23, 24, 25, 26) au contenant de préparation (3") dans le dispositif (1").

6. Utilisation selon l'une quelconque des revendications 1 à 5, dans laquelle le contenant de préparation (3', 3") présente un ensemble mélangeur (18, 18").

7. Utilisation d'un procédé comprenant les étapes suivantes :
a) l'agencement d'un contenant de préparation (3, 3', 3") pré-conditionné avec des ingrédients dans un logement (2) d'un dispositif (1, 1', 1"),
b) l'amenée d'un fluide (10) d'un contenant de stockage (4, 4") dans le contenant de préparation (3, 3") par l'intermédiaire d'une conduite d'amenée (6),
c) la préparation des ingrédients à l'aide du fluide (10, 10") de manière à obtenir un mélange de substances (11, 11'), et
d) l'évacuation du mélange de substances (11, 11') du contenant de préparation (3, 3', 3") par l'intermédiaire d'une conduite d'évacuation (12, 12', 12") pour le faire passer dans un contenant de réception (13, 13', 13"), pour la préparation de mélanges de substances pharmaceutiques ou biopharmaceutiques (11, 11").

8. Utilisation selon la revendication 7 pour la préparation de médicaments adaptés individuellement à un patient.

9. Utilisation selon l'une quelconque des revendications 7 à 8, dans laquelle, après l'insertion du contenant de préparation (3), un codage (16) du contenant de préparation (3) est lu par une unité de lecture (17) et les paramètres nécessaires à la mise en oeuvre du procédé sont interrogés dans une mémoire et fournis à une unité de contrôle et de commande (5) .

10. Dispositif (1, 1', 1"), constitué d'un contenant de préparation (3, 3', 3") pouvant être agencé dans un logement (2) et renfermant des ingrédients, d'un contenant de stockage (4, 4") renfermant un fluide (10, 10"), qui peut être amené de façon régulée au contenant de préparation (3, 3', 3") par l'intermédiaire d'une conduite d'amenée (6), et d'un contenant de réception, qui communique avec le contenant de préparation (3, 3', 3") par l'intermédiaire d'une conduite d'évacuation (12, 12', 12"), **caractérisé en ce que**, pour la préparation de mélanges de substances pharmaceutiques ou biopharmaceutiques (11, 11"), un filtre stérile (22), ou un filtre antiviral ou une combinaison d'un filtre stérile et d'un filtre antiviral est monté (e) en amont ou est monté (e) en aval du contenant de préparation (3, 3'ou 3").

11. Dispositif selon la revendication 11, **caractérisé en ce que** le contenant de préparation (3, 3', 3") présente au moins un filtre stérile (22) ou un filtre antiviral une combinaison d'un filtre stérile et d'un filtre antiviral.

12. Dispositif selon la revendication 11 ou 12, **caractérisé en ce que** le contenant de préparation (3, 3', 3") est réalisé sous la forme d'un tampon sur ou dans lequel est agencé (e) un filtre stérile ou un filtre antiviral ou une combinaison d'un filtre stérile et d'un filtre antiviral.

13. Dispositif selon l'une quelconque des revendications 11 à 13, **caractérisé en ce que** le contenant de préparation (3, 3', 3") et le contenant de réception (13, 13', 13") peuvent être soudés l'un à l'autre par l'intermédiaire d'une liaison par tuyaux flexibles.

14. Dispositif selon l'une quelconque des revendications 11 à 14, **caractérisé en ce que** le contenant de préparation (3, 3', 3") et/ou le contenant de réception (13, 13', 13") présentent au moins un capteur destiné à déterminer un paramètre du mélange de substances préparé.
